# EUROPÄISCHE PATENTSCHRIFT

(11) **EP 2 106 759 B1**
(45) Veröffentlichungstag und Bekanntmachung des Hinweises auf die Patenterteilung: **15.03.2017**
(21) Anmeldenummer: 09156427.8
(22) Anmeldetag: 27.03.2009
(51) Int. Cl.: A61B 17/29, A61B 17/00

(54) **Medizinisches Instrument mit flexiblem Einsatz**
Medical instrument with flexible insert
Instrument médical doté d'une utilisation flexible

(30) Priorität: 31.03.2008 DE 102008017298
(43) Veröffentlichungstag der Anmeldung: 07.10.2009
(73) Patentinhaber: Karl Storz GmbH & Co. KG, 78532 Tuttlingen (DE)
(72) Erfinder: Bacher, Uwe, 78532, Tuttlingen (DE); Zahler, Sabine, 85591 Vaterstetten (DE)
(74) Vertreter: Witte, Weller & Partner Patentanwälte mbB

(56) Entgegenhaltungen:
- WO-A-95/13023
- WO-A-99/15089
- DE-A1- 4 332 497
- DE-A1- 19 820 486
- DE-U1- 9 401 556
- DE-U1- 29 701 170
- US-A- 5 630 832
- US-A- 6 077 287
- US-A1- 2003 236 549
- US-A1- 2004 230 221

## Beschreibung

Die Erfindung betrifft ein medizinisches Instrument mit einem flexiblen Schaft, der distalseitig ein abwinkelbares Ende und einen sich längs des flexiblen Schaftes erstreckenden flexiblen Einsatz aufweist, an dessen distalem Ende ein Werkzeug angeordnet ist und der proximal ein Verbindungsstück aufweist, welches mit einem beweglichen Griffteil lösbar verbunden ist, das an einer am proximalen Ende des flexiblen Schaftes angeordneten Handhabe um eine Schwenkachse verschwenkbar angebracht ist, wobei der flexible Einsatz proximalseitig an einer abseits der Längsachse des Schaftes gelegenen Stelle mit dem Griffteil gelenkig verbunden ist.

Ein derartiges medizinisches Instrument ist aus der US 6 077 287 A bekannt.

Derartige medizinische Instrumente in Form von Präparier- und Fasszangen, sowie in Form von Scheren, Stanzen und Zangen für die Probeexcision, sind aus dem Katalog der Anmelderin Laparoskopie, 5. Ausgabe 1/2005 aus den Kapiteln 4 und 5 bekannt. Diese medizinischen Instrumente bestehen aus den drei Hauptbauteilen Schaft, Handhabe mit beweglichem Griffteil und dem in den Schaft eingeschobenen Einsatz. Am distalen Ende trägt der Einsatz ein Werkzeug, das je nach Anwendungsgebiet ausgestaltet ist, also z.B. als Greifteile bei Präparier- und Fasszangen, als Schneiden bei Scheren, Stanzen und Zangen für die Probeexcision. Das Werkzeug kann als Elektrode ausgebildet sein und zur Koagulation dienen. Das proximale Ende des Einsatzes ist lösbar mit dem beweglichen Griffteil verbunden. Die Bewegung des Griffteiles verursacht ein axiales Hin- und Herverschieben des Betätigungselementes des Einsatzes und verursacht bspw. ein Öffnen oder Schließen von Maulteilen des Werkzeuges. Der Einsatz wird üblicherweise so montiert, dass er von distal in den Schaft eingeschoben wird, bis ein Anschlag am Werkzeug an das distale Ende des Schaftes läuft. Meist über eine Schraubverbindung, wird der Einsatz am proximalen Ende des Schaftes ortsfest befestigt. Der über das proximale Ende des Schaftes vorstehende axial verschiebbare Teil des Einsatzes dient als Verbindungsstück zur Verbindung mit dem Griffteil. Bei starren Einsätzen hat sich die sog. "Click-Line-Verbindungstechnik" (siehe DE 197 220 62) etabliert, dabei wird ein kugelförmiges Ende des Verbindungsstückes in eine Pfanne im beweglichen Griffteil in einer extremen Verschwenkstellung des Griffteiles eingeschoben, das dann anschließend in die eigentliche Arbeitsstellung verschwenkt wird, in der dann das kugelige Ende vor einem Austreten aus der Pfanne gesperrt ist.

Bei medizinischen Instrumenten mit einem flexiblen Schaft hat sich gezeigt, dass durch die Krümmung eines langen Schaftes und eines ggf. abwinkelbaren Endes des Schaftes, axiale Lageverschiebungen zwischen dem Schaft und dem darin aufgenommenen Einsatz erfolgen, mit der Folge, dass der Einsatz etwas axial aus dem proximalen Ende herausgeschoben wird. Dies kann so erklärt werden, dass der Einsatz trotz seiner Flexibilität eine gewisse Steifigkeit aufweisen muss, um die in axialer Richtung wirkenden Kräfte zum Öffnen und Schließen der Maulteile übertragen zu können. Aufgrund der Tatsache, dass der hohle Schaft diesen Einsatz umgibt, ist dessen Krümmung zwangsläufig etwas unterschiedlich zu der Krümmung des darin aufgenommenen Einsatzes, wodurch sich diese, wenn auch geringfügigen, axialen Verschiebungen ergeben, die unerwünscht sind. Bei extremen Verschiebungen wäre eine sichere Handhabung des Werkzeuges nicht mehr gewährleistet.

Bei Geräten mit einem flexiblen Schaft und einer zusätzlichen Abwinklung des Schaftes ist es notwendig im Schaft Steuerseile anzubringen, die für die Abwinklung des Schaftes sorgen. Diese Steuerseile müssen am proximalen Ende des Schaftes herausgeführt und an entsprechende Steuerelemente angebracht werden. Daher hat es sich bei flexiblen Schäften etabliert, das proximale Ende des Einsatzes, also das Verbindungsstück mit dem beweglichen Griffteil, nicht direkt in der Längsachse des Schaftes zur Verbindungsstelle zu führen, sondern zu einer abseits gelegenen Stelle (siehe US 6 077 287 A). Daraus ergeben sich Probleme im Bereich der Verbindungsstelle des proximalen Ende des Einsatzes mit dem Griffteil dahingehend, dass beim Bewegen des beweglichen Griffteiles, gewisse Ausweich- oder Schwenkbewegungen des proximalen Endbereiches des Einsatzes erforderlich sind, da hier ein Längenausgleich notwendig werden kann. Dies führt letztendlich dazu, dass ein Schieben dieses proximalen Endabschnittes des Einsatzes dazu führt, dass dieser distal aus dem Schaft herausbewegt wird.

Es ist daher Aufgabe der vorliegenden Erfindung hier Abhilfe zu schaffen und für eine betriebssichere Befestigung des Einsatzes zu sorgen.

Erfindungsgemäß wird die Aufgabe dadurch gelöst, dass der flexible Einsatz am distalen Ende des flexiblen Schafts axial unverrückbar befestigt ist, und dass der flexible Einsatz in einem Bereich zwischen einer Stelle an der dieser in dem Schaft eintritt und der Verbindung mit dem beweglichen Griffteil freiliegend verläuft, und dass die Stelle, an der das Verbindungsstück des flexiblen Einsatzes in eine Öffnung am beweglichen Griffteil einbringbar ist, in einer Ebene liegt, die durch die Ebene einer ringförmigen Fingeraufnahme des Griffteiles aufgespannt ist, und dass sich seitlich versetzt von der Öffnung ein Abschnitt des Griffteiles in Richtung der Schwenkachse erstreckt, so dass der freiliegende Bereich des flexiblen Einsatzes neben diesem versetzten Abschnitt verläuft.

Die Maßnahme der distalseitigen Befestigung des Einsatzes am flexiblen Schaft sorgt dafür, dass das Werkzeug, also das über das proximale Ende des Schaftes hinausstehende Teil des Einsatzes, nicht mehr axial aus dem Schaft herausbewegt werden kann.

Die gelenkige Verbindung des Einsatzes proximalseitig mit dem Griffteil, erlaubt beim Verschwenken des Griffteiles Ausweichbewegungen des Abschnittes zwischen der Anlenkstelle am Griffteil und der Eintrittsstelle in den Schaft.

Beide Maßnahmen kombiniert, führen zu einer optimalen Lagefixierung des Einsatzes, wenngleich schon jede Einzelmaßnahme für sich gesehen, einen bemerkenswerten Beitrag für die Lagefixierung als solche leistet.

In einer weiteren Ausgestaltung der Erfindung ist die distale Befestigung des Einsatzes am flexiblen Schaft eine Schraubbefestigung.

Diese Maßnahme hat den Vorteil, dass durch eine konstruktiv einfache und fertigungstechnisch einfach herzustellende Schraubverbindung die sichere Lagefixierung bewerkstelligt werden kann. Dies ist auch einfach zu lösen, um das medizinische Instrument zu zerlegen und zu reinigen.

In einer weiteren Ausgestaltung der Erfindung ist die distale Befestigung des Einsatzes am flexiblen Schaft eine Rastbefestigung.

Diese ist handhabungstechnisch sehr einfach, der Einsatz muss lediglich soweit von distal in den Schaft eingeschoben werden, bis die Verrastung eingreift. Zum Zerlegen des Instruments ist es dann notwendig, dass die Rastverbindung gelöst werden kann.

In einer weiteren Ausgestaltung der Erfindung ist das proximalseitige Verbindungsstück kugelförmig ausgestaltet.

Diese an sich bekannte Maßnahme hat den Vorteil, dass die gelenkige Verbindung mit dem beweglichen Griffteil dadurch einfach und sicher hergestellt werden kann.

In einer weiteren Ausgestaltung der Erfindung ist das Verbindungsstück mittels einer Sperre im Griffteil gehalten.

Diese Maßnahme hat den Vorteil, dass die Sperre dafür sorgt, dass das Verbindungsstück unverlierbar am Griffteil gehalten ist, dennoch aber die notwendige gelenkige Bewegung erlaubt.

In einer weiteren Ausgestaltung der Erfindung ist die Sperre aus einem sperrenden Eingriff mit dem Verbindungsstück in eine nicht sperrende Position bewegbar.

Diese Maßnahme hat den Vorteil, dass durch ein Bewegen der Sperre die Verbindung zwischen dem proximalen Ende des Einsatzes und dem Griffteil bewerkstelligt bzw. gelöst werden kann.

In einer weiteren Ausgestaltung der Erfindung ist die Sperre ein axialer, quer zur Längsrichtung des medizinischen Instruments verlaufender Stift, der eine Aussparung aufweist, durch die das Verbindungsstück in der nicht sperrenden Stellung bewegbar ist.

Diese Maßnahme hat zum einen den Vorteil, dass ein solcher Stift ergonomisch anzuordnen und demzufolge der Verbindungs- bzw. der Lösvorgang einfach durchzuführen ist, nämlich durch Verschieben des Stiftes. Durch Vorsehen der Aussparung kann in einer Stellung das Verbindungsstück an der Sperre vorbeibewegt werden, in der anderen Stellung ist dies gehindert, so dass das kugelförmige Ende nicht über die Sperre hinausbewegt und somit vom Griffteil abgenommen werden kann.

In einer weiteren Ausgestaltung weist das bewegliche Griffteil eine Öffnung auf, über die das Verbindungsstück einschiebbar ist.

Diese Maßnahme hat den Vorteil, dass die Montage sehr einfach und zielgerichtet durchgeführt werden kann, nämlich indem das proximale Ende des Einsatzes, also das Verbindungsstück, in die Öffnung im beweglichen Griffteil eingeschoben wird, wobei sich die Sperre in ihrer nicht sperrenden Stellung befindet und dann durch Verschieben der Sperre die sperrende Verbindung bewerkstelligt wird.

In einer weiteren Ausgestaltung der Erfindung ist die Sperre durch eine Halterung in dem Griffteil gehalten und wird durch eine Federung gegen diese Halterung vorgespannt.

Diese Maßnahme hat den Vorteil, dass die Sperre in eine ganz bestimmte Stellung gedrückt wird, sinnvollerweise in die sperrende Stellung, und dort gehalten ist. Durch die vorspannende Federkraft ist sichergestellt, dass die Sperre immer in diese sperrende Stellung gedrückt wird.

In einer weiteren Ausgestaltung der Erfindung weist die Sperre einen aus der Halterung vorstehenden Knopf auf, über den die Sperre von der Außenseite her bewegbar ist.

Diese Maßnahme hat den Vorteil, dass von der Außenseite her die Sperre über den Knopf eingedrückt werden kann und somit in die nicht sperrende Stellung gebracht werden kann, in der das Verbindungsstück des Einsatzes nunmehr entweder eingeschoben oder abgezogen werden kann, je nachdem, ob es sich um einen Montage- oder einen Zerlegungsvorgang handelt. Danach wird der Knopf wieder freigegeben und die Sperre wird dann automatisch in die sperrende Stellung gedrückt, so dass dann bei eingeschobenem Verbindungsteil dieses unverlierbar im Griffteil gesperrt aufgenommen ist.

In einer weiteren Ausgestaltung der Erfindung ist ein distal vor dem Verbindungsstück angeordneter durchmessergeringerer Abschnitt vorhanden, der in eine mit der Öffnung in Verbindung stehende Nut einführbar ist und über diese seitlich aus dem Griffteil herausführbar ist, wobei das Verbindungsstück größer ist als die Breite der Nut.

Diese Maßnahme hat nun den erheblichen Vorteil, dass über diese seitliche Nut der durchmessergeringere Abschnitt seitlich aus dem Griffteil herausgeführt werden kann. Ein Heraustreten des durchmessergrößeren Verbindungsstücks aus dieser seitlichen Nut ist gesperrt, so dass sichergestellt ist, dass das proximale Ende des Einsatzes nicht aus dieser seitlichen Nut aus dem Griffteil heraustreten kann. Dennoch erlaubt die Nut Verschwenkbewegungen des Einsatzes längs der Nut, so dass die erforderlichen Relativbewegungen zwischen Griffteil und proximal vom Schaft vorstehenden Endabschnitt des Einsatzes möglich sind. Durch die kugelkopfartige Ausgestaltung des Verbindungsstückes wird dies zusätzlich noch gefördert, gleichzeitig ist aber eine sichere Halterung durch die Sperre gewährleistet.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
Die Erfindung wird nachfolgend anhand einiger ausgewählter Ausführungsbeispiele und weiteren Beispielen, welche nicht Teil der vorliegenden Erfindung sind, in Zusammenhang mit den beiliegenden Zeichnungen näher beschrieben und erläutert. Es zeigen:
   Fig. 1 ein medizinisches Instrument mit abwinkelbaren Schaft in Seitenansicht,
   Fig. 1a das medizinische Instrument von Fig. 1 aus proximaler Betrachtung, d.h. aus Sicht des Operateurs,
   Fig. 1b eine perspektivische Ansicht des medizinischen Instrumentes,
   Fig. 2 eine ausschnittsweise Seitenansicht des medizinischen Instruments von

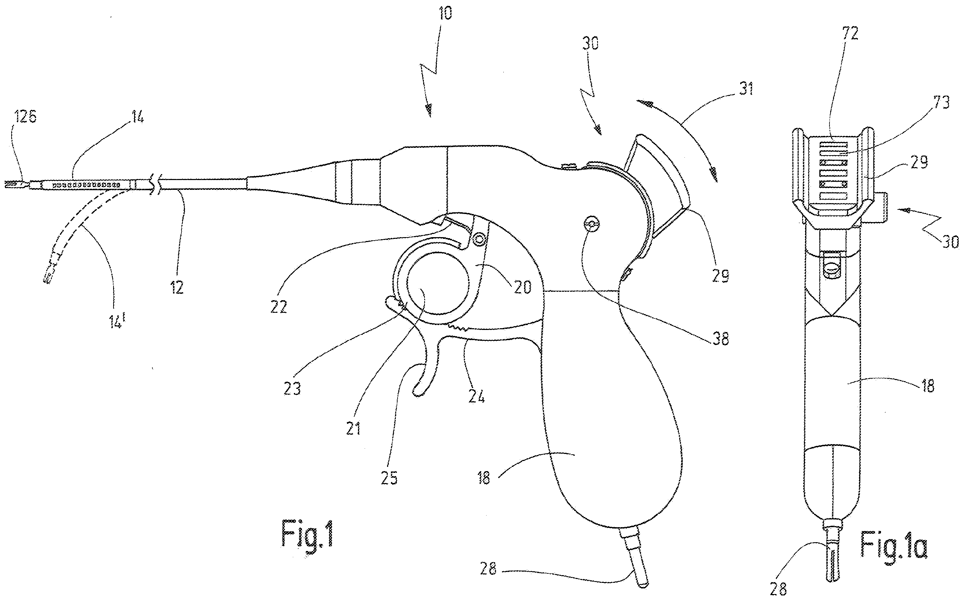

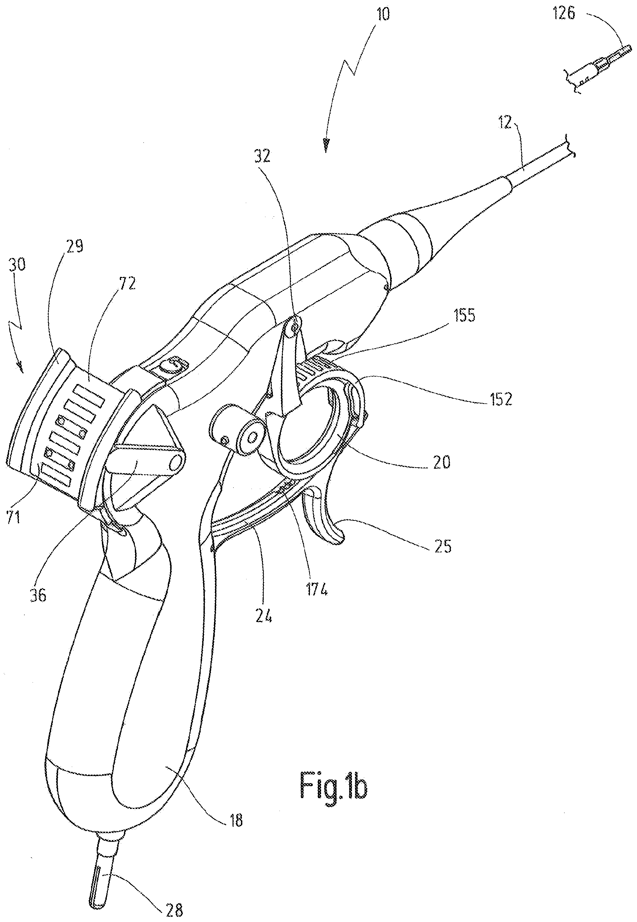

   Fig. 1 von der gegenüberliegenden Seite,

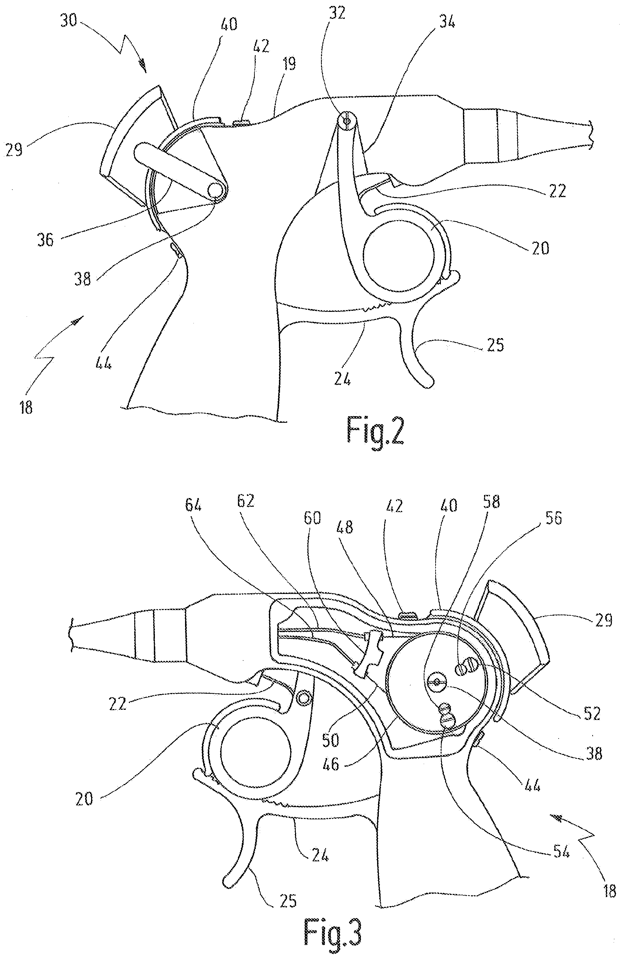

   Fig. 3 eine ausschnittsweise Seitenansicht des medizinischen Instruments von Fig. 1 mit geöffnetem Gehäuse einer Handhabe,

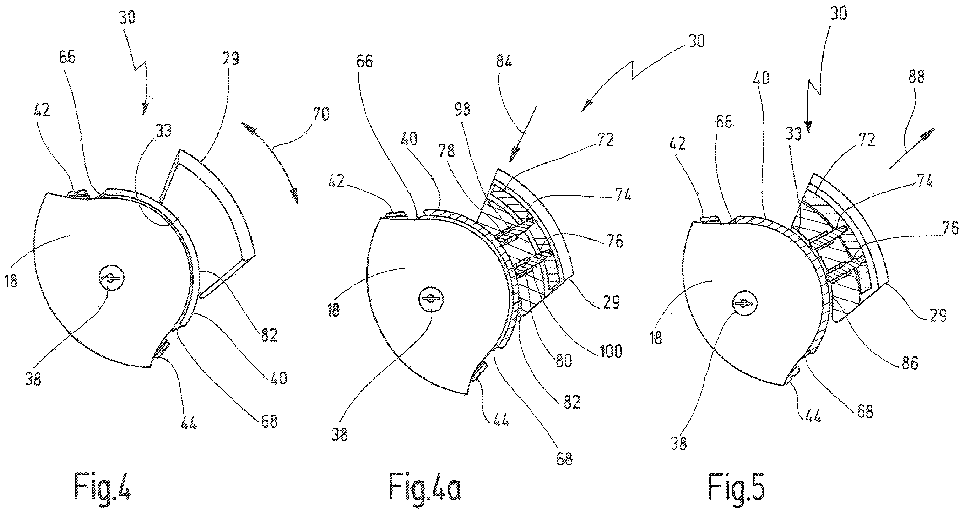

   Fig. 4 eine ausschnittsweise Detailansicht der Handhabe zur Erläuterung der beispielhaften Arretierung einer beispielhaften Abwinkelsteuerung,
   Fig. 4a die Detailansicht von Fig. 4 mit Schnittansicht eines Steuerelements der
   Abwinkelsteuerung in arretiertem Zustand,
   Fig. 5 die Detailansicht wie Fig. 4a, in nicht arretiertem Zustand,

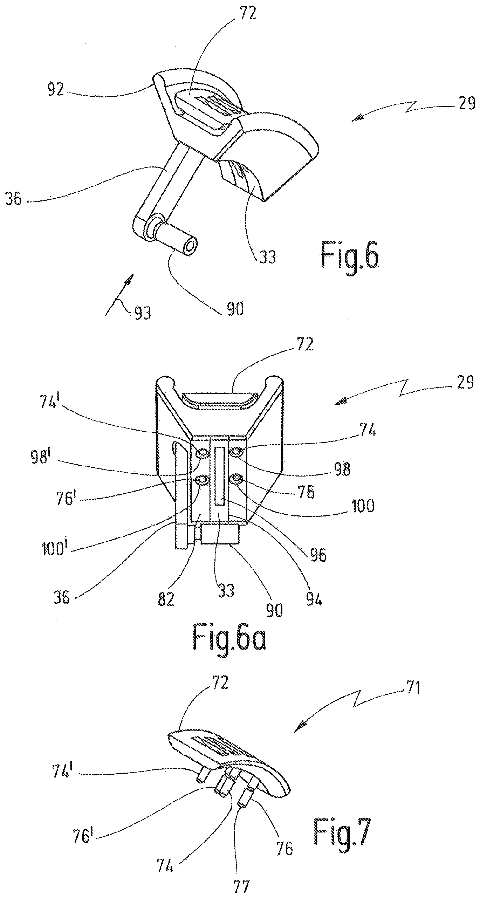

   Fig. 6 das Steuerelement der Abwinkelsteuerung in perspektivischer Einzelansicht,
   Fig. 6a eine Ansicht des Steuerelements von Fig. 6, entlang des Pfeils 93 von Fig. 6,
   Fig. 7 ein Betätigungselement der Abwinkelsteuerung in perspektivischer Einzelansicht,

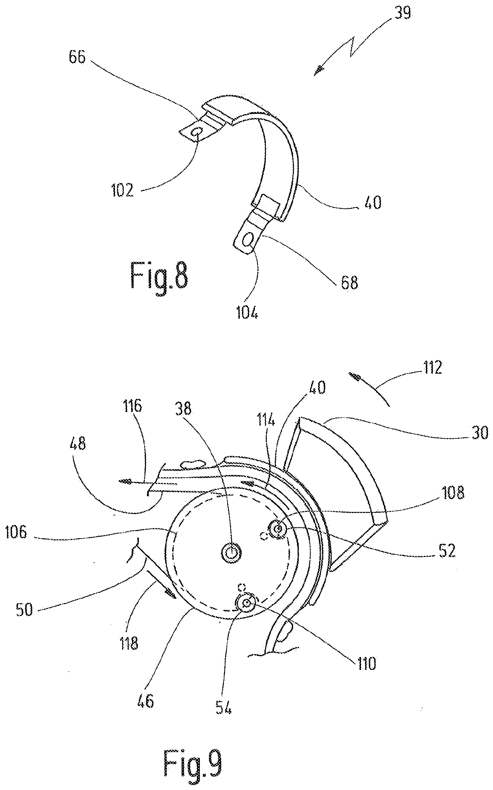

   Fig. 8 ein Reibungselement zur Arretierung der Abwinkelsteuerung in perspektivischer Einzelansicht,
   Fig. 9 eine Detailansicht wie in Fig. 4 dargestellt, mit Verlauf von Steuerseilen um eine Trommel,

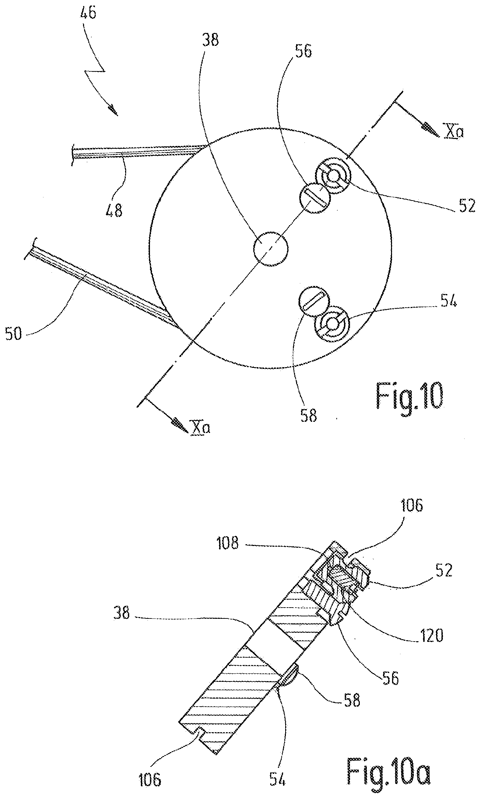

   Fig. 10 eine Seitenansicht der Trommel von Fig. 9,
   Fig. 10a einen Schnitt längs der Linie Xa-Xa in Fig. 10,

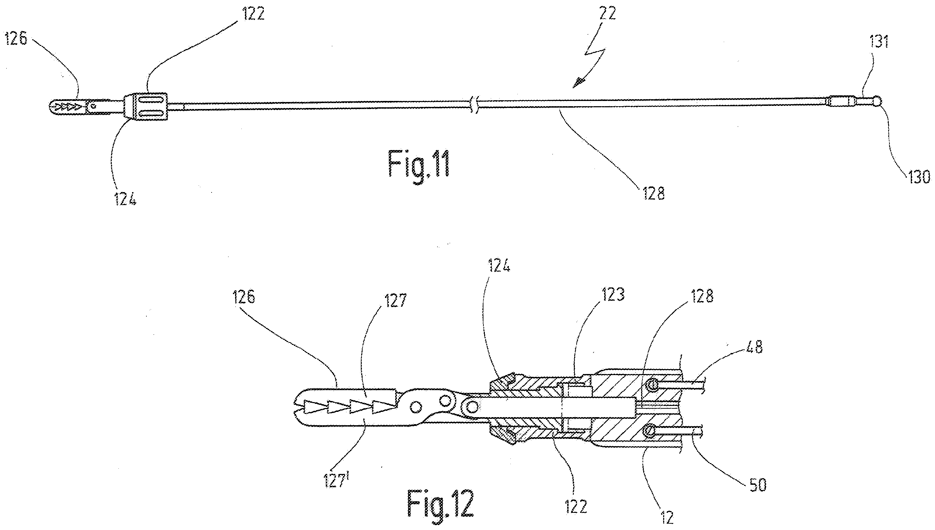

   Fig. 11 eine Seitenansicht eines erfindungsgemäßen Werkzeugeinsatzes für das medizinische Instrument von Fig. 1,
   Fig. 12 eine vergrößerte Detailansicht des distalen Endes des Werkzeugs von Fig. 11,

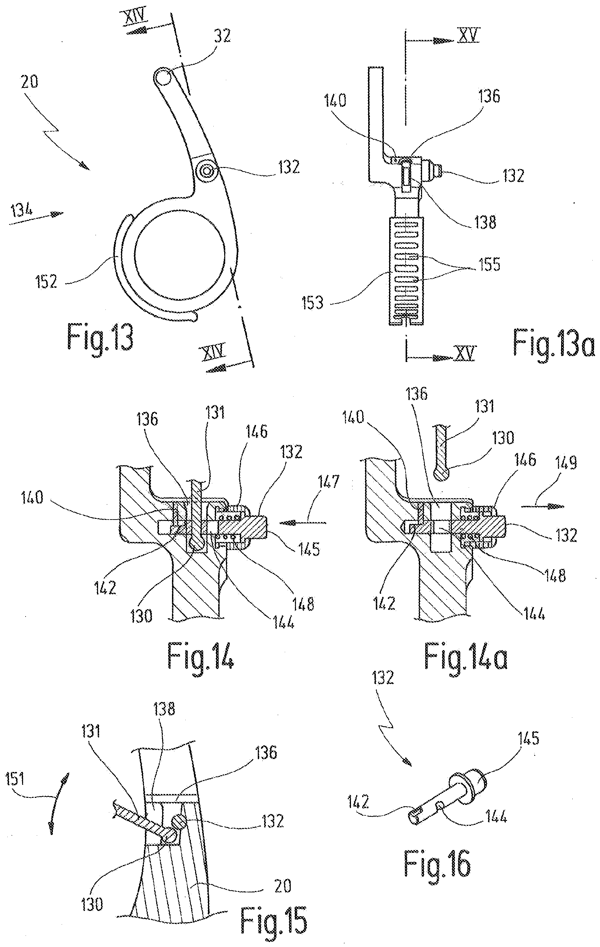

   Fig. 13 Seitenansicht eines Griffteils des in Fig. 1 gezeigten Instruments,
   Fig. 13a eine Ansicht des erfingungsgemäßen Griffteils in Richtung des Pfeils 134 von Fig. 13 betrachtet,
   Fig. 14 ein vergrößerter abschnittsweiser Schnitt längs der Linie XIV-XIV in Fig. 13, mit eingehängtem Ende des Werkzeugeinsatzes von Fig. 11 im gesperrten Zustand,
   Fig. 14a eine der Fig. 14 entsprechende Darstellung mit freigegebenem Ende des Werkzeugeinsatzes,
   Fig. 15 ein vergrößerter abschnittsweiser Schnitt längs der Linie XV-XV in
   Fig. 13a, mit eingehängtem Ende des Werkzeugeinsatzes von Fig. 11, Fig. 16 eine Sperre in perspektivischer Einzelansicht,

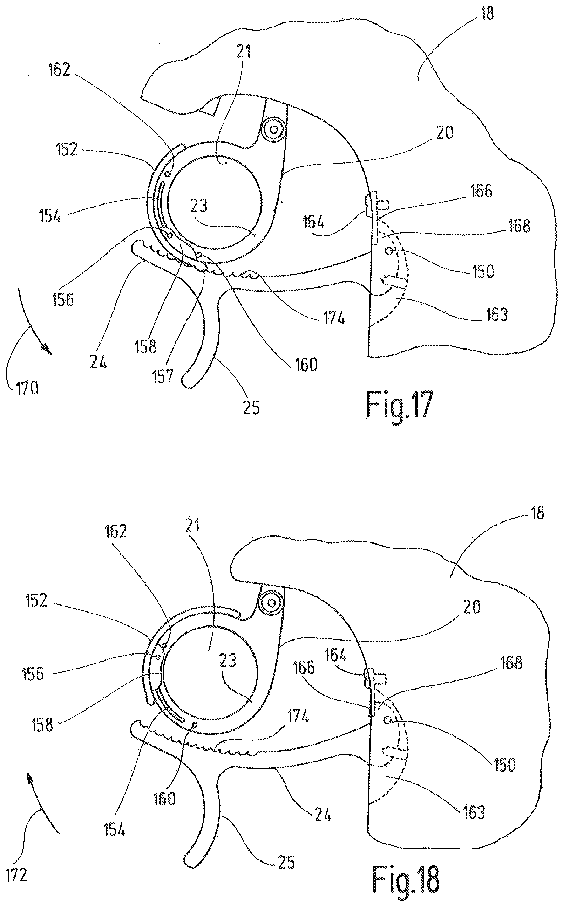

   Fig. 17 eine Detailansicht eines medizinischen Instruments im Bereich des Griffteils zur Veranschaulichung einer beispielhaften Rastverbindung,
   Fig. 18 eine Darstellung entsprechend Fig. 17, mit gelöster Rastverbindung,

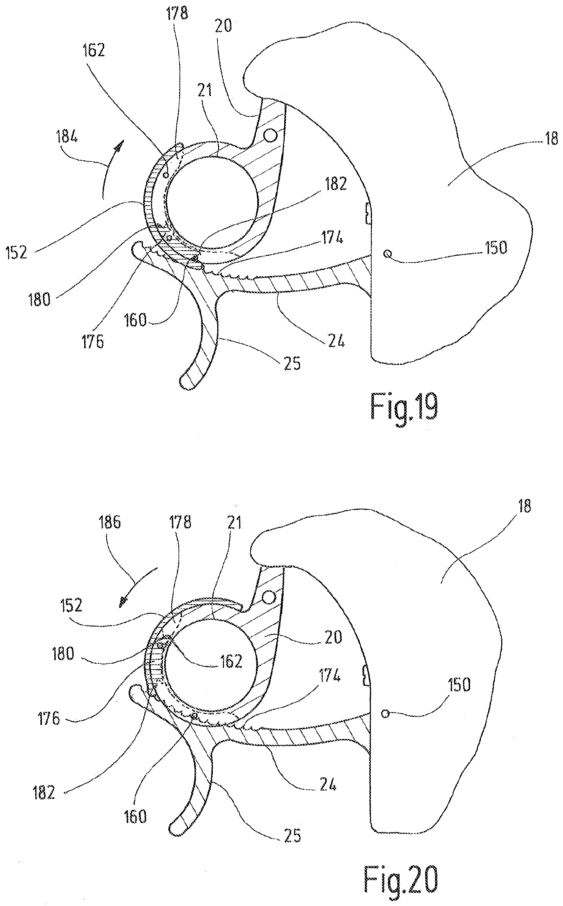

   Fig. 19 eine Darstellung entsprechend Fig. 17, als Schnitt in der Betrachtungsebene und mit deaktivierter Rastverbindung, und
   Fig. 20 eine Darstellung entsprechend Fig. 19, mit aktivierter Rastverbindung.

Ein in den Figuren dargestelltes medizinisches Instrument ist in seiner Gesamtheit mit der Bezugsziffer 10 bezeichnet.

Das in Fig. 1 gezeigte medizinische Instrument 10 weist einen flexiblen Schaft 12 auf, an dessen distalen Ende sich ein abwinkelbarer Bereich 14 befindet. Am Bereich 14 ist distalseitig ein Werkzeug 126 angeordnet. Das Werkzeug 126 stellt ein distales Ende eines in Fig. 11 dargestellten Einsatzes 22 dar. Das proximale Ende des Schaftes 12 ist mit einer Handhabe 18 verbunden.

Die Handhabe 18 ihrerseits weist ein bewegliches Griffteil 20 auf. Dieses besitzt eine runde Öffnung 21, die von einem Ringabschnitt 23 umgrenzt ist, durch die vorzugsweise der Zeigefinger des Operateurs geführt werden kann, um eine Bewegung des Griffteils 20, das um die in der Fig. 2 gezeigte Schwenkachse 32 verschwenkbar ist, durchzuführen. Das Griffteil 20 ist mit dem proximalen Ende des Einsatzes 22 verbunden. Durch die Verbindung des Griffteils 20 mit dem Einsatz 22, steht dieser in Wirkverbindung mit dem Werkzeug 126 und dient so zu dessen Betätigung, so z.B. einem Öffnen und Schließen eines Maulteils.

Weiterhin kann das Griffteil 20 in Kontakt mit einer Raste 24 gebracht werden, die eine ungewollte Bewegung des Griffteils 20 in eine distale Richtung verhindern kann. Um eine Lösung dieser beispielhaften Rastverbindung zu ermöglichen, weist die Raste 24 unter anderem einen bogenförmigen Ansatz 25 auf, der ein Verschwenken der Raste 24 durch den Operateur, vorzugsweise mit dem Mittelfinger, entsprechend der Beschreibung in Zusammenhang mit den Figuren 17ff. ermöglicht.

Des Weiteren ist an der Handhabe 18 ein Steuerelement 29 einer beispielhaften Abwinkelsteuerung 30 angeordnet, durch dessen Bewegung entsprechend der Richtungen des Doppelpfeils 31 um eine Drehachse 38, die senkrecht zur hier dargestellten Achse des Schaftes 12 steht, die Abwinklung des abwinkelbaren Endes 14 des Schaftes 12 gesteuert werden kann. Ein Beispiel für eine Richtung der Abwinklung ist in Fig. 1 durch das abgewinkelte Ende 14' dargestellt.

In Fig. 1a blickt man auf das Steuerelement 29 der beispielhaften Abwinkelsteuerung 30 und ein sich darauf befindliches Betätigungselement 71 in Form eines Drückers 72. Dieser kann durch einen Daumen des Operateurs betätigt werden, wodurch eine Bewegung des Steuerelements 29 ermöglicht wird. Zur besseren Greifbarkeit sind hierfür Rillen 73 auf dem Drücker 72 angebracht.

Das Instrument 10 weist ferner noch einen Stromanschluss 28 auf, der z.B. zur Stromversorgung von optional einsetzbaren Koagulationseinsätzen dienen kann.

Aus Fig. 2 kann man den Bereich der Verschwenkbarkeit des Griffteils 20 um die Schwenkachse 32 im Bereich einer Aussparung 34 erkennen. Das Steuerelement 29 ist über einen Verbindungsarm 36 mit der Drehachse 38 verbunden.

Zwischen einem Gehäuse 19 der Handhabe 18 und dem Steuerelement 29 befindet sich ein Reibungselement 39 in Form eines Reibungsbleches 40, welches durch Schrauben 42 und 44 an der Außenseite der Handhabe 18 befestigt ist. Dieses Reibungsblech 40 dient, wie im Folgenden noch näher beschrieben wird, die Abwinkelsteuerung 30 in einer bestimmten Position zu arretieren.

Eine in Fig. 3 erkennbare Trommel 46 ist auf der Drehachse 38 befestigt und steht somit durch den Verbindungsarm 36 mit dem Steuerelement 29 der beispielhaften Abwinkelsteuerung 30 in Wirkverbindung. Eine entsprechende Betätigung des Steuerelements 29 hat somit auch eine direkte Bewegungsübertragung auf die Trommel 46 zur Folge. An der Trommel 46 enden zwei vom abwinkelbaren Ende 14 des Schaftes 12 durch den Schaft 12 verlaufende Steuerseile 48 und 50, die jeweils abseits der Drehachse 38 verlaufen und in diesem Ausführungsbeispiel durch Befestigungsschrauben 52 und 54 in Kombination mit Sicherungsschrauben 56 und 58 an der Trommel 46 befestigt sind. Dazu werden die Steuerseile 48 und 50 von distal her aus Hüllen 62 und 64 austretend durch eine Führung 60 zu der Trommel 46 geleitet. Die Steuerseile 48 und 50 sind die Betätigungselemente für das abwinkelbare Ende 14. Zusammen mit der Trommel 46 und dem Verbindungsarm 36 ergibt sich somit die Wirkverbindung zwischen dem Steuerelement 29 der Abwinkelsteuerung 30 und dem abwinkelbaren Ende 14. Eine detailliertere Funktionsbeschreibung erfolgt später im Zusammenhang mit Fig. 9.

In Zusammenhang mit den Figuren 4 bis 8 wird nunmehr auf die Arretierbarkeit der Abwinkelsteuerung 30 und somit des abwinkelbaren Endes 14 des Schaftes 12 mit Hilfe des Reibungsblechs 40 näher eingegangen.

In Fig. 4 sieht man, dass sich eine Unterseite 33 des Steuerelements 29 in direktem Kontakt mit dem Reibungsblech 40 befindet, welches über abgewinkelte Federbleche 66 und 68 und mittels der Schrauben 42 und 44 an der Handhabe 18 befestigt ist. Das Reibungsblech 40 verläuft somit im Abstand zur Außenseite der Handhabe 18 an der es montiert ist. Eine Bewegung der des Steuerelements 29 um die Drehachse 38 entsprechend der Richtungen des Doppelpfeils 70, wird hierbei durch den reibenden Kontakt zwischen dem Steuerelement 29 und dem Reibungsblech 40 an einer Reibkontaktfläche 82 verhindert, bzw. gebremst.

In Fig. 4a ist zu erkennen, dass die beispielhafte Abwinkelsteuerung 30 den Drücker 72 umfasst.

Dieser ist, wie auch in Fig. 1b zu erkennen ist, proximalseitig für den Operateur gut zugänglich. Vom Drücker 72 stehen Stifte 74, 74', 76, 76' (siehe auch Fig. 7) vor, die distalseitig durch Hülsen 78, 80 im Körper des Steuerelements 29 geführt und gehalten sind. Die Spitzen der Stifte stehen auf dem Reibungsblech 40 direkt auf und ergeben somit eine Wirkverbindung zwischen dem Drücker 72 und dem Reibungsblech 40. Durch Eindrücken des Drückers 72 entsprechend der Richtung des Pfeils 84, werden die Stifte durch Bohrungen 98, 100 im Körper des Steuerelements 29 axial bewegt, wobei sie das Reibungsblech 40 in Richtung der Handhabe 18 drücken. Das Reibungsblech 40 bewegt sich dadurch von der Unterseite 33 des Steuerelements 29 weg. Die Reibkontaktfläche 82 ist damit gelöst und es ergibt sich ein Spalt 86, wie er in Fig. 5 dargestellt ist. Die für diese Positionsänderung des Reibungsblechs 40 benötigte Flexibilität, wird vor allem durch die Federbleche 66 und 68, aber auch durch Langlochöffnungen 102 und 104, wie sie in Fig. 8 gezeigt sind, ermöglicht.

Die durch Betätigen des Drückers 72 resultierende Stellung, wie sie in Fig. 5 gezeigt ist, erlaubt nun eine reibungsarme Bewegung des Steuerelements 29, wie sie in Fig. 4 anhand des Doppelpfeils 70 gezeigt ist.

Lediglich die Spitzen der vier Stifte 74, 74', 76, 76' stehen auf dem Reibungsblech 40 und gleiten reibungsarm über dessen Oberfläche. Dazu können diese z.B. aus einem reibungsarmen Kunststoffmaterial bestehen. Es kann auch ein metallischer Grundkörper mit dem reibungsarmen Material überzogen sein, oder an einen metallischen Stumpf eine reibungsarme Spitze ausgesetzt sein.

Nimmt nun der Operateur seinen Finger, vorzugsweise den Daumen, wieder vom Drücker 72, so wird aufgrund der Spannung durch die Federbleche 66 und 68 das Reibungsblech 40 wieder gegen die Unterseite 33 des Steuerelements 29 der Abwinkelsteuerung 30 gedrückt, so dass der Spalt 86 verschwindet und wieder die Reibkontaktfläche 82 vorliegt. Entsprechend erfahren die Stifte 74, 74', 76, 76' und damit der Drücker 72 auch wieder eine proximal gerichtete Bewegung entsprechend der Richtung des Pfeils 88. Dadurch wird die Abwinkelsteuerung 30 wieder in ihrer Position arretiert. Dies ist somit im Verschwenkbereich des Steuerelements 29 stufenlos durchzuführen.

Das Steuerelement 29 der Abwinkelsteuerung 30 ist in Fig. 6, 6a und 7 näher dargestellt, wobei man hier deutlich den Drücker 72 und dessen proximale Zugänglichkeit erkennt. Der Drücker 72 ist dabei an einem Fingermuldenteil 92 befestigt, welches über den Verbindungsarm 36 und mit einem Achsbolzen 90 an der Drehachse 38 montiert wird.

Fig. 6a zeigt die Unterseite 33, die mit dem Reibungsblech 40 in Kontakt kommt. In diesem Ausführungsbeispiel ist der Drücker 72 mit vier Stiften 74, 74', 76 und 76' ausgestattet, die axial in den Bohrungen 98, 98', 100 und 100' verlaufen und bewegbar sind. Zwischen den beiden Stiftpaaren 74, 76 und 74', 76' ist ein Kunststoffinlet 96 angeordnet, welches durch ein Halteblech 94 am Fingermuldenteil 92 befestigt wird. Dieses Kunststoffinlet 96 dient dazu, die Reibung zwischen dem Steuerelement 29 und dem Reibungsblech 40 zu erhöhen und somit die Arretierung in der gewünschten Position zu stärken.

In Fig. 7 ist der Drücker 72 mit den vier Stiften 74, 74', 76 und 76' gut zu erkennen. Aufgrund ihrer distal abgerundeten Spitzen 77 wird die Reibung bei ihrem Gleiten auf dem Reibungsblech 40 auf ein Minimum reduziert, was die Bedienbarkeit der Abwinkelsteuerung 30 erleichtert.

Das in Fig. 8 gezeigte Beispiel des Reibungsblechs 40 ist an den gegenüberliegenden Enden mit den abgewinkelten Federblechen 66 und 68 verbunden, die beide eine Langlochöffnung 102 bzw. 104 besitzen, wodurch eine Beweglichkeit des Reibungsblechs 40 an der Handhabe 18, entsprechend der vorherigen Beschreibungen, also auf die Handhabe 18 zu bzw. von dieser weg, ermöglicht wird. Die Abwinklungen an den Federblechen 66 und 68 sorgen für die entsprechende Andrückkraft und somit für die entsprechend feste Arretierung zwischen Steuerelement 29 und Handhabe 18 an der das Reibungsblech 40 montiert ist.

Anhand der Fig. 9 bis Fig. 10a soll die Funktionsweise der beispielhaften Abwinkelsteuerung 30 weiter erläutert und die Befestigung der Steuerseile 48 und 50 an der Trommel 46 beschrieben werden.

In Fig. 9 ist der Verlauf der Steuerseile 48 und 50 in der Trommel 46 dargestellt. Letztere umfasst eine umfängliche Rille 106 in der die Steuerseile 48 und 50 geführt werden, um dann in Bohrungen 108 und 109 der Befestigungsschrauben 52 und 54 zu enden. An diesen sind die Steuerseile 48 und 50 dann fest montiert.

Wird nun das Steuerelement 29 in Richtung des Pfeils 112 bewegt, so erfährt die Trommel 46, durch die zuvor beschriebene Wirkverbindung über den Verbindungsarm 36, eine Drehbewegung um die Drehachse 38, wie sie durch die Richtung des Pfeils 114 angegeben ist. Für die an der Trommel 46 befestigten Steuerseile 48 und 50 bedeutet dies, dass sie ebenfalls eine Bewegung erfahren und zwar wird das Steuerseil 48 in Richtung des Pfeils 116 in den Schaft 12 hineingeschoben und das Steuerseil 50 entsprechend in Richtung des Pfeils 118 aus dem Schaft herausgezogen. Aufgrund der bereits erwähnten Wirkverbindung zwischen den Steuerseilen 48 und 50 mit dem abwinkelbaren Ende 14, wird dieses in seiner Winkelstellung folglich verändert. Dies resultiert in einem Abwinkeln entsprechend der in Fig. 1 durch das abwinkelbare Ende 14' dargestellten Form.

Die entgegengesetzte Bewegung führt wieder zu einem Strecken des Schaftes 12 bzw. einem Abwinkeln, aus der Sicht von Fig. 1, nach oben. Durch Freigabe des Drückers 72 kann die Position bzw. Abwinklung des abwinkelbaren Endes 14 in jeder beliebigen Stellung arretiert werden.

Die Anordnung der Trommel und die des Steuerelements um 90° verdreht, würde anstatt der in Fig. 1 dargestellten Abwinkelebene "oben-unten" eine um die Schaftachse um 90° verdrehte Abwinkelebene "links-rechts" ergeben. Man kann die Steuerseile auch vertauscht anbringen, dann führt z.B. ein Verschieben des Steuerelements "nach vorne" anstatt zu einer Abwinklung nach "unten" zu einer Abwinklung nach "oben".

In Fig. 10a erkennt man die Rille 106. Des Weiteren sieht man die Bohrung 108 der Befestigungsschraube 52. Durch diese wird in dem hier erwähnten Beispiel das Steuerseil 48 in die Befestigungsschraube 52 eingeführt und mittels einer Fixierschraube 120 in dieser Befestigungsschraube fest montiert. Entsprechendes gilt für die hier nicht im Querschnitt gezeigte Befestigungsschraube 54 und das Steuerseil 50. Anschließend kann dann die Länge der Steuerseile 48 und 50 durch individuelle Drehung der Schrauben 52 und 54 eingestellt werden. In einem Ausführungsbeispiel haben diese dazu unterschiedlich verlaufende Gewinde, so dass Befestigungsschraube 52 ein Rechts- und Befestigungsschraube 54 ein Linksgewinde aufweist. Nach erfolgter Einstellung der Steuerseile 48 und 50 erfolgt dann die Fixierung der Befestigungsschrauben 52 und 54 mittels der Sicherungsschrauben 56 und 58. Diese verhindern eine eigenständige Drehung der Befestigungsschrauben 52 und 54 und somit ein ungewolltes Verstellen der Steuerseile 48 und 50.

In den folgenden Figuren 11 bis 16 wird die Ausgestaltung und die Montage des erfindungsgemäßen Einsatzes 22 beschrieben.

Der in Fig. 11 gezeigte Einsatz 22 weist distal ein Werkzeug 126, hier zwei spreizbare Maulteile 127, 127' auf, welches über ein stabförmiges flexibles Betätigungselement 128 mit einem Verbindungsstück 130 in Wirkverbindung steht. Proximal hinter dem Werkzeug 126 ist eine Haube 124 und ein Schraubverschluss 122 angebracht, die beide zur Befestigung des Einsatzes 22 an einem flexiblen Schaft, wie z.B. an den flexiblen Schaft 12 aus Fig. 1, dienen. Das proximale Ende des Einsatzes 22 weist, wie zuvor bereits erwähnt, das Verbindungsstück 130 auf, welches bspw. zur Befestigung an dem Griffteil 20 des medizinischen Instruments 10 dient. Dazu ist dieses in diesem Ausführungsbeispiel das Ende kugelförmig ausgestaltet und proximal hinter einem durchmessergeringerem Abschnitt 131 am Einsatz 22 angeordnet.

Fig. 12 zeigt die Befestigung des distalen Endes des Einsatzes 22 am distalen Ende des Schafts 12. Dabei ist die proximal hinter dem Werkzeug 126 befindliche Haube 124 fest mit dem Einsatz 22 verbunden. Dadurch wird ein Verrutschen des Schraubverschlusses 122 in eine distale Richtung verhindert. Dieser Schraubverschluss 122 wird seinerseits dann auf ein Außengewinde 123 am distalen Ende des Schaftes 12 aufgeschraubt. Das Kraftübertragungselement 128 wird dazu zuvor von distal in den Schaft 12 eingeführt. Durch diese Befestigung ist das distale Ende des Einsatzes 22 lagefixiert. Ein Abwinkeln des abwinkelbaren Endes 14 verursacht dann nicht mehr ein Ausschieben des Einsatzes 22 aus dem distalen Ende des Schafts 12.

Die Fig. 13 bis 16 zeigen das erfindungsgemäße Griffteil 20, dessen Schwenkachse 32 und einer Sperre 132 zum lösbaren Verbinden mit dem proximalen Ende des Einsatzes 22. In Fig. 13a erkennt man eine in Richtung der Schwenkachse 32 mündende Öffnung 136 durch die das kugelförmige Ende des Verbindungsstücks 130 eingeführt wird. Der distal folgende durchmessergeringe Abschnitt 131 am Einsatz 22 kann über eine Nut 138 seitlich aus dem Inneren des Griffteils 20 herausgeführt werden (siehe Fig. 15). Um das Ende des Einsatzes 22 einzuführen, muss eine Sperre 132 gedrückt werden, so dass das Verbindungsstück 130 diese passieren kann. Dies lässt sich an den Figuren 14 und 14a erkennen.

Die in Fig. 16 ersichtliche Sperre 132 wird durch eine Halterung 146 am Griffteil 20 gehalten. Durch eine Feder 148 wird diese ferner gegen den Rand dieser Halterung 146 gedrückt. Die in Fig. 14 gezeigte Position stellt somit die Grundstellung der Sperre 132 dar. Man sieht hierbei, wie das Verbindungsstück 130 aufgrund seines hier kugelförmigen Endes durch die Sperre 132 blockiert wird und somit nicht mit Bezug auf die Darstellung nach oben durch die Öffnung 136 gelangen kann. Betätigt man nun die Sperre 132 entgegen der Richtung, in der sie durch die Feder 148 gedrückt wird, also in Richtung des Pfeiles 147, so bewegt sich eine Aussparung 144 an der Sperre 132, die sich in der Darstellung von Fig. 14 rechts des Verbindungsstückes 130 befindet, in eine zentrale Position der Öffnung 136, wie sie bspw. in Fig. 14a gezeigt ist. Dieses Eindrücken kann über einen Knopf 145 durchgeführt werden, der seitlich über die Halterung 146 hinaus zur Außenseite ragt. Diese Aussparung 144 gibt dem kugelförmigen Verbindungsstück 130 gerade genügend Platz, um an dieser Sperre 132 vorbeizugelangen. Dadurch kann das Verbindungsstück 130 durch die Öffnung 136 hindurch aus der Halterung in dem Grillteil 20 entfernt werden. Gibt man die Sperre 132 wieder frei, so bewegt sich diese entsprechend der Richtung des Pfeils 149 aus Fig. 14a wieder nach außen. Ursache hierfür ist wiederum die Feder 148. Gleichzeitig verschiebt sich dabei dann auch die Aussparung 144.

Will man das Verbindungsstück 130 nun wieder in die Halterung des Griffteils 20 einsetzen, so bedarf es wieder einem Eindrücken der Sperre 132 in Richtung des Pfeils 147 aus Fig. 14, so dass die Aussparung 144 wieder in die zentrale Lage, wie sie in Fig. 14a gezeigt ist, zu liegen kommt. Auf diese Weise kann man nun das kugelförmige Ende wieder an der Sperre 132 vorbeiführen und das Verbindungsstück 130 durch die Öffnung 136 am Griffteil 20 befestigen.

In Fig. 15 sieht man mit einem Verbindungsstück 130 veranschaulicht, wie sich dieses unterhalb der Sperre 132 befindet. Eine Bewegung nach oben ist nicht möglich. Der durchmessergeringe Abschnitt 131 am proximalen Ende des Einsatzes 22 passt durch die Nut 138 hindurch und somit ist eine Bewegbarkeit entsprechend des Doppelpfeils 151 ermöglicht wird. Diese Bewegungsfreiheit wird bei der Bewegung des Griffteils 20 benötigt.

Um ein Verdrehen der um die Längsachse drehbaren Sperre 132 und damit auch ein Verdrehen der Aussparung 144 zu verhindern, ist am distalen Ende der Sperre 132 eine axiale Nut 142 eingearbeitet. Diese dient auch als Anschlag für die Verschiebebewegung. Dies ist in den Figuren 14 und 14a, sowie auch in der perspektivischen Darstellung der Fig. 16 gezeigt. In dieser Nut 142 endet nun bei der Befestigung im Griffteil 20 ein Stift 140, der zwar ein unerwünschtes Drehen um die Längsachse der Sperre 132 verhindert, eine axiale Beweglichkeit der Sperre 132 in Richtung der Pfeile 147 und 149 jedoch erlaubt.

In den Figuren 17 bis 20 ist eine beispielhafte Rastverbindung, die sich durch die Raste 24 auf das Griffteil 20 ergibt, näher erläutert.

Die Raste 24 ist an einer Schwenkachse 150 verschwenkbar an der Handhabe 18 in einer Aussparung 163 angebracht. Durch Kontakt mit dem Griffteil 20 kann diese Raste 24 in diesem Ausführungsbeispiel die Bewegung des Griffteils 20 in die distale Richtung unterbinden. Dazu wird die Raste 24 durch die Vorspannung aufgrund eines Federblechs 166 in Richtung des Griffteils 20 gedrückt.

Die Raste 24 weist dazu an der dem Griffteil 20 zugewandten Seite Rastzähne 174 auf, die mit einem Raststift 160 am Griffteil 20 in Eingriff kommen. Die Neigung der Flanken der Rastzähne 174 in Richtung Handhabe 18, erlauben eine Bewegung des Griffteils 20 in Richtung der Handhabe 18, sperren diese jedoch in der entgegengesetzten Richtung.

Will man die beispielhafte Rastverbindung kurzzeitig lösen, so verschwenkt man die Raste 24 entsprechend der Richtung des Pfeils 170, vorzugsweise durch Betätigung am bogenförmigen Ansatz 25, was zu einer Endstellung, wie sie in Fig. 18 gezeigt ist, führt. Aufgrund der Vorspannung wird die Raste 24 bei Loslassen dieser entsprechend der Richtung des Pfeils 172 wieder zurück an den Griffteil 20 gebracht.

Um für einen bestimmten Zeitraum die Rastverbindung zu deaktivieren, ist am Griffteil 20 eine Rastsperre 152 angebracht.

Die Rastsperre 152 ist als ein gebogenes Element, im dargestellten Ausführungsbeispiel als ein gebogener Streifen 153 ausgebildet (siehe auch Fig. 13a) dessen Krümmung der Krümmung der Außenseite des Ringabschnittes 23 des Griffteils 20 angepasst ist.

Aussparungen bzw. Austanzungen 155 im Streifen 153 erhöhen die Griffigkeit.

Diese Rastsperre 152 kann, wie aus Fig. 17 ersichtlich, zwischen Griffteil 20 und Raste 24 gebracht werden. In diesem Fall ist die Rastverbindung deaktiviert und das Griffteil 20 ist frei in beide Richtungen bewegbar. Dazu weist die Rastsperre 152 eine abgerundete Backe 157 auf, die in beiden Richtungen über die Zähne 174 laufen kann. Dies entspricht einer zweiten Stellung der Rastsperre 152. Um die Rastverbindung nun wieder zu aktivieren, kann die Rastsperre 152 in Richtung eines Raststifts 162 geschoben werden. Dies entspricht einer ersten Stellung der Rastsperre 152. Auf beiden Seiten des Streifens 153 ist eine Abdeckung 158 vorhanden. Diese deckt einen in der Rastsperre quer verlaufenden Führungsstift 156 ab, der in beidseitigen Führungsnuten 154 im Ringabschnitt 23 läuft. Die Abdeckungen 158 selbst können über hier nicht näher dargestellte Stifte an der Rastsperre 152 befestigt werden. Dementsprechend durchläuft die Rastsperre 152 eine kreisförmige Bewegung, wie sie durch die Form des Ringabschnitts 23 des Griffteils 20 vorgegeben ist und endet somit in einer Position, wie sie in Fig. 18 gezeigt ist. Dadurch liegt dann ein zuvor durch die Rastsperre 152 blockierter Raststift 160 frei und kann mit den Zähnen 174 der Raste 24 in Eingriff treten.

Wie in den Figuren 19 und 20 gezeigt ist, ist umfänglich im Griffteil 20 eine äußere Nut 178 eingeschnitten. In dieser ist eine in radialer Richtung des Ringabschnittes 23 vorspringende Stufe 176 der Rastsperre 152 bewegbar, die mittig an der Rastsperre 152 angeordnet ist. An jeweils einem Ende dieser Stufe 176 befinden sich Federclips 180 bzw. 182. Diese können entsprechend der Position der Rastsperre 152 in den Raststift 160 bzw. 162 einrasten und damit ein einfaches Hin- und Herrutschen der Rastsperre 152 verhindern. Diese wird somit in den jeweiligen Stellungen gehalten.

Fig. 19 zeigt in diesem Zusammenhang die zweite Stellung der Rastsperre 152, in der die Rastverbindung deaktiviert ist. Der Federclip 182 der Stufe 176 an der Rastsperre 152 ist in den Raststift 160 eingerastet, und blockiert somit den Kontakt zwischen den Rastzähnen 174 und dem Raststift 160. Eine Bewegung der Rastsperre 152 in Richtung des Pfeils 184 würde schließlich in der ersten Stellung enden, wie sie in Fig. 20 gezeigt ist. Dabei ist der Federclip 180, der sich an der Stufe 176 befindet, in den Raststift 162 eingerastet, und damit ist die Rastsperre 152 in dieser Position fixiert. Der Raststift 160 liegt somit frei und kann in die Zähne 174 der Raste 24 einhaken.

Dahingegen ist eine proximale Bewegung des Griffteils 20, die z.B. zu einem Schließen der Maulteile 127, 127' führen würde, über die Raste 24 weiter möglich.

Eine Deaktivierung der Rastverbindung kann nun wieder durch Bewegung der Sperre 152 analog zu dem zuvor gesagten entsprechend der Richtung des Pfeils 186, vorzugsweise nach vorherigem Absenken der Raste 24, gemäß der Beschreibung zu den Figuren 17 und 18, erfolgen.

Die Handhabungsperson kann das Instrument 10, wie in Fig. 1a ersichtlich, über die Handhabe 18 ergreifen. Mit dem Daumen kann der Drücker 72 gedrückt werden und dann kann das Steuerelement 29 verschoben werden. Dies bewirkt ein entsprechendes Abwinkeln des abwinkelbaren Endes 14 des Schafts. Ein Freigeben des Drückers 72 arretiert das abwinkelbare Ende 14 in der entsprechenden Stellung.

Ein Bewegen des Griffteils 20, z.B. mit dem eingeschobenen Zeigerfinger, erlaubt das Öffnen und Schließen der Maulteile 127, 127' über den Einsatz 22 in jeder beliebigen Abwinkelstellung des abwinkelbaren Endes 14 des Schaftes 12.

Bei deaktivierter Rastfunktion ist die Bewegung des Griffteils 20 in beiden Schwenkrichtungen möglich.

Bei aktivierter Rastfunktion kann diese kurzzeitig durch Verschwenken der Raste 24 mit dem Mittelfinger über den bogenförmigen Ansatz 25 bewerkstelligt werden.

Die Handhabungsperson kann somit auf sehr ergonomische Art und Weise das medizinische Instrument 10 einfach und sicher bedienen.

## Patentansprüche

1. Medizinisches Instrument mit einem flexiblen Schaft (12), der distalseitig ein abwinkelbares Ende (14) und einen sich längs des flexiblen Schaftes (12) erstreckenden flexiblen Einsatz (22) aufweist, an dessen distalem Ende ein Werkzeug (126) angeordnet ist und der proximal ein Verbindungsstück (130) aufweist, welches mit einem beweglichen Griffteil (20) lösbar verbunden ist, das an einer am proximalen Ende des flexiblen Schaftes (12) angeordneten Handhabe (18) um eine Schwenkachse (32) verschwenkbar angebracht ist, wobei der flexible Einsatz (22) proximalseitig an einer abseits der Längsachse des Schaftes (12) gelegenen Stelle mit dem Griffteil (20) gelenkig verbunden ist, **dadurch gekennzeichnet, dass** der flexible Einsatz (22) am distalen Ende des flexiblen Schaftes (12) axial unverrückbar befestigt ist, und dass der flexible Einsatz (22) in einem Bereich zwischen einer Stelle an der dieser in dem Schaft (12) eintritt und der Verbindung mit dem beweglichen Griffteil (20) freiliegend verläuft, und dass die Stelle, an der das Verbindungsstück (130) des flexiblen Einsatzes (22) in eine Öffnung (136) am beweglichen Griffteil (20) einbringbar ist, in einer Ebene liegt, die durch die Ebene einer ringförmigen Fingeraufnahme des Griffteils (20) aufgespannt ist, und dass sich seitlich versetzt von der Öffnung (136) ein Abschnitt des Griffteiles (20) in Richtung der Schwenkachse (32) erstreckt, so dass der freiliegende Bereich des flexiblen Einsatzes (22) neben diesem versetzten Abschnitt verläuft.

2. Medizinisches Instrument nach Anspruch 1, **dadurch gekennzeichnet, dass** die distale Befestigung des Einsatzes (22) am flexiblen Schaft (12) eine Schraubbefestigung ist.

3. Medizinisches Instrument nach Anspruch 1, **dadurch gekennzeichnet, dass** die distale Befestigung des Einsatzes (22) am flexiblen Schaft (12) eine Rastbefestigung ist.

4. Medizinisches Instrument nach einem der Ansprüche 1 bis 3, **dadurch gekennzeichnet, dass** das Verbindungsstück (130) kugelförmig ausgestaltet ist.

5. Medizinisches Instrument nach einem der Ansprüche 1 bis 4, **dadurch gekennzeichnet, dass** das Verbindungsstück (130) mittels einer Sperre (132) im Griffteil (20) gelenkig haltbar ist.

6. Medizinisches Instrument nach Anspruch 5, **dadurch gekennzeichnet, dass** die Sperre (132) aus dem sperrenden Eingriff mit dem Verbindungsstück (130) in eine nicht sperrende Position bewegbar ist.

7. Medizinisches Instrument nach Anspruch 6, **dadurch gekennzeichnet, dass** die Sperre (132) ein axialer, quer zur Längsrichtung des medizinischen Instruments (10) verlaufender Stift ist, der eine Aussparung (144) aufweist, durch die das Verbindungsstück (130) in der nicht sperrenden Stellung bewegbar ist.

8. Medizinisches Instrument nach einem der Ansprüche 1 bis 7, **dadurch gekennzeichnet, dass** das bewegliche Griffteil (20) eine Öffnung (136) aufweist, über die das Verbindungsstück in das Griffteil (20) einschiebbar ist.

9. Medizinisches Instrument nach einem der Ansprüche 5 bis 7, **dadurch gekennzeichnet, dass** die Sperre (132) durch eine Halterung (146) am Griffteil (20) gehalten ist und durch eine Federung (148) gegen diese Halterung (146) vorgespannt ist.

10. Medizinisches Instrument nach Anspruch 9, **dadurch gekennzeichnet, dass** die Sperre (132) einen aus der Halterung (146) vorstehenden Knopf (145) aufweist, über den die Sperre von der Außenseite her bewegbar ist.

11. Medizinisches Instrument nach einem der Ansprüche 4 bis 10, **dadurch gekennzeichnet, dass** ein distal vor dem Verbindungsstück (130) angeordneter durchmessergeringerer Abschnitt (131) des Einsatzes (22) in eine mit der Öffnung (136) in Verbindung stehende Nut (138) einführbar ist und über diese Nut (138) seitlich aus dem Griffteil (20) herausführbar ist, wobei das Verbindungsstück (130) größer ist als die Breite der Nut (138).

12. Medizinisches Instrument nach einem der Ansprüche 1 bis 11, **dadurch gekennzeichnet, dass** die Sperre (132) eine axiale Nut (142) aufweist, in die ein im Griffteil (20) angeordneter Stift führbar ist.

## Claims

1. Medical instrument with a flexible shaft (12) comprising a bendable distal end (14) and, extending along the flexible shaft (12), a flexible insert (22) which comprises, arranged at its distal end, a tool (126) and which comprises, at its proximal end, a connection piece (130) connected releasably to a movable grip part (20), which is mounted to a handle (18), arranged at the proximal end of the flexible shaft (12), so as to be pivotable about a pivot axis (32), wherein the flexible insert (22) is connected in an articulated manner to the grip part (20) at the proximal end at a position that is remote from the longitudinal axis of the shaft (12), **characterized in that** the flexible insert (22) is fastened in an axially immovable manner at the distal end of the flexible shaft (12), and that the flexible insert (22) extends exposed in a region between a position at which it enters the shaft (12) and the connection with the movable grip part (20), and that the position at which the connection piece (130) of the flexible insert (22) is insertable in an opening (136) at the movable grip part (20) is located in a plane that is spanned by a plane of an annular finger holder of the grip part (20), and that a section of the grip part (20) extends laterally offset from the opening (136) towards the pivot axis (32), so that the exposed region of the flexible insert (22) extends alongside this displaced section.

2. Medical instrument according to Claim 1, **characterized in that** the fastening of the distal end of the insert (22) to the flexible shaft (12) is a screw fastening.

3. Medical instrument according to Claim 1, **characterized in that** the fastening of the distal end of the insert (22) to the flexible shaft (12) is a lock mechanism.

4. Medical instrument according to any of Claims 1 to 3, **characterized in that** the connection piece (130) has a spherical shape.

5. Medical instrument according to any of Claims 1 to 4, **characterized in that** the connection piece (130) can be held in an articulated manner in the grip part (20) by means of a catch (132).

6. Medical instrument according to Claim 5, **characterized in that** the catch (132) is movable from the locking engagement with the connection piece (130) to a non-locking position.

7. Medical instrument according to Claim 6, **characterized in that** the catch (132) is an axial pin extending transverse to the longitudinal direction of the medical instrument (10) and comprising a recess (144) through which the connection piece (130) can be moved to the non-locking position.

8. Medical instrument according to any of Claims 1 to 7, **characterized in that** the movable grip part (20) comprises an opening (136) via which the connection piece can be pushed into the grip part (20).

9. Medical instrument according to any of Claims 5 to 7, **characterized in that** the catch (132) is held on the grip part (20) by a retainer (146) and is biased against this retainer (146) by a spring (148).

10. Medical instrument according to Claim 9, **characterized in that** the catch (132) has a knob (145) which protrudes beyond the retainer (146) and via which the catch is movable from the outside.

11. Medical instrument according to any of Claims 4 to 10, **characterized in that** a portion (131) of the insert (22), that is arranged distally in front of the connection piece (130) and comprises a smaller diameter, is insertable in a groove (138) connected to the opening (136), and is arranged to be guided laterally out of the grip part (20) via this groove (138), wherein the connection piece (130) is larger than the width of the groove (138).

12. Medical instrument according to one of Claims 1 to 11, **characterized in that** the catch (132) comprises an axial groove (142) into which a pin arranged in the grip part (20) is insertable.

## Revendications

1. Instrument médical doté d'une tige flexible (12), qui présente côté distal une extrémité pouvant être courbée (14) et un insert flexible (22) s'étendant le long de la tige flexible (12), dont l'extrémité distale porte un outil (126) et qui présente côté proximal une pièce de liaison (130), qui est assemblée de façon séparable à une partie de saisie mobile (20), qui est montée de façon pivotante autour d'un axe de pivotement (32) sur une poignée (18) disposée à l'extrémité proximale de la tige flexible (12), dans lequel l'insert flexible (22) est relié côté proximal de façon articulée à la partie de saisie (20) en un point situé à l'écart de l'axe longitudinal de la tige (12), **caractérisé en ce que** l'insert flexible (22) est fixé de façon axialement immobile à l'extrémité distale de la tige flexible (12), et **en ce que** l'insert flexible (22) s'étend librement dans une région située entre un point où celui-ci pénètre dans la tige (12) et la liaison avec la partie de saisie mobile (20), et **en ce que** le point où la pièce de liaison (130) de l'insert flexible (22) peut être introduite dans une ouverture (136) sur la partie de saisie (20) est situé dans un plan, qui passe par le plan d'un logement de doigt annulaire de la partie de saisie (20), et **en ce qu'**une partie de la partie de saisie (20) latéralement décalée de l'ouverture (136) s'étend en direction de l'axe de pivotement (32), de telle manière que la région libre de l'insert flexible (22) s'étende à côté de cette partie décalée.

2. Instrument médical selon la revendication 1, **caractérisé en ce que** la fixation distale de l'insert (22) à la tige flexible (12) est une fixation vissée.

3. Instrument médical selon la revendication 1, **caractérisé en ce que** la fixation distale de l'insert (22) à la tige flexible (12) est une fixation par encliquetage.

4. Instrument médical selon l'une quelconque des revendications 1 à 3, **caractérisé en ce que** la pièce de liaison (130) est de forme sphérique.

5. Instrument médical selon l'une quelconque des revendications 1 à 4, **caractérisé en ce que** la pièce de liaison (130) est maintenue de façon articulée dans la partie de saisie (20) au moyen d'un dispositif de verrouillage (132).

6. Instrument médical selon la revendication 5, **caractérisé en ce que** le dispositif de verrouillage (132) est déplaçable de l'engagement de verrouillage avec la pièce de liaison (130) à une position non verrouillée.

7. Instrument médical selon la revendication 6, **caractérisé en ce que** le dispositif de verrouillage (132) est une broche axiale s'étendant transversalement à la direction longitudinale de l'instrument médical (10), et qui présente une découpe (144), à travers laquelle la pièce de liaison (130) peut être déplacée dans la position non verrouillée.

8. Instrument médical selon l'une quelconque des revendications 1 à 7, **caractérisé en ce que** la partie de saisie mobile (20) présente une ouverture (136), par laquelle la pièce de liaison peut être introduite dans la partie de saisie (20).

9. Instrument médical selon l'une quelconque des revendications 5 à 7, **caractérisé en ce que** le dispositif de verrouillage (132) est maintenu sur la partie de saisie (20) par une attache (146) et est précontraint contre l'attache par des ressorts (148).

10. Instrument médical selon la revendication 9, **caractérisé en ce que** le dispositif de verrouillage (132) présente un bouton (145) saillant hors de l'attache (146), par lequel le dispositif de verrouillage peut être déplacé depuis le côté extérieur.

11. Instrument médical selon l'une quelconque des revendications 4 à 10, **caractérisé en ce qu'**une partie de plus petit diamètre (131) de l'insert (22) disposée en position distale devant la pièce de liaison (130) peut être introduite dans une rainure (138) se trouvant en communication avec l'ouverture (136) et peut être retirée latéralement hors de la partie de saisie (20) au moyen de cette rainure (138), dans lequel la pièce de liaison (130) est plus grande que la largeur de la rainure (138).

12. Instrument médical selon l'une quelconque des revendications 1 à 11, **caractérisé en ce que** le dispositif de verrouillage (132) présente une rainure axiale (142), dans laquelle une broche disposée dans la partie de saisie (20) peut être menée.
